# EUROPEAN PATENT APPLICATION

(11) **EP 4 563 691 A1**
(43) Date of publication of application: **04.06.2025**
(21) Application number: 23846587.6
(22) Date of filing: 26.07.2023
(51) Int. Cl.: C12M 1/34

(54) **CELL STATE ESTIMATION SYSTEM**

(30) Priority: 27.07.2022 JP 2022119292
(71) Applicant: Integriculture Inc., Tokyo 113-0033 (JP)
(72) Inventor: KAWASHIMA, Ikko, Tokyo 113-0033 (JP)
(74) Representative: Calysta NV
(86) International application number: PCT/JP2023/027440
(87) International publication number: WO 2024/024857

(57) **Abstract**

Provided is a cell state estimation system capable of accurately determining a state of a cell to be cultured. The cell state estimation system includes: an appearance information acquisition portion that acquires appearance information of a second cell different from a first cell to be cultured, in which at least a part of a culture medium providing a growth environment to the first cell provides a growth environment to the second cell; a physical property measurement portion that measures at least one physical property of at least one of the culture medium that has provided a growth environment of the second cell and the second cell; and an estimation reference information portion that stores estimation reference information referred to for estimating a state of at least one of the first cell and the culture medium, in which the estimation of the state of at least one is based on at least one of a result acquired by the appearance information acquisition portion and a result measured by the physical property measurement portion.

## Description

### Technical Field

The present invention relates to a system for estimating a state of a cell to be cultured.

### Background Art

A system for determining a state of a cell to be cultured from an image obtained by imaging the cell is known (see, for example, Patent Literature 1). In this system, a cell cultured in a cell culture apparatus is imaged and determined.

### Citation List

### Patent Literature

Patent Literature 1: WO 2014/203322 A

### Summary of Invention

### Technical Problem

The cells are immersed in the culture medium in a tank in which the cells are cultured. The culture medium is a medium that provides a growth environment and is essential for culturing cells. The culture medium is often in a cloudy state in the growth process, and the cells could not be clearly imaged through the cloudy culture medium. For this reason, it has been difficult to accurately determine the state of the cell.

The present invention has been made in view of the above points, and an object thereof is to provide a system capable of accurately determining the state of a cell to be cultured.

### Solution to Problem

A feature of the cell state estimation system according to the present invention is to include:
an appearance information acquisition portion that acquires appearance information of a second cell different from a first cell to be cultured, in which at least a part of a culture medium providing a growth environment to the first cell provides a growth environment to the second cell;
a physical property measurement portion that measures at least one physical property of at least one of the culture medium that has provided a growth environment of the second cell and the second cell; and
an estimation reference information portion that stores estimation reference information referred to for estimating a state of at least one of the first cell and the culture medium, in which the estimation of the state of at least one is based on at least one of a result acquired by the appearance information acquisition portion and a result measured by the physical property measurement portion.

### Advantageous Effects of Invention

The state of a cell to be cultured can be accurately determined.

### Brief Description of Drawings

[Fig. 1] Fig. 1 is a block diagram that illustrates an example of a cell state estimation system according to the present embodiment.
[Fig. 2] Fig. 2 is a functional block diagram that illustrates a configuration of a control device 330 of the cell state estimation system according to the present embodiment.
[Fig. 3] Fig. 3 is a flowchart that illustrates a state determination processing of a product cell by the control device 330.
[Fig. 4] Fig. 4 is a table that illustrates an example of a state space table for estimation.
[Fig. 5] Fig. 5 is a plan view that schematically illustrates a holding member 510 according to the present embodiment.
[Fig. 6] Fig. 6 is a cross-sectional view taken along line A-A' of the holding member 510 according to the present embodiment.
[Fig. 7] Fig. 7 is a plan view that schematically illustrates a holding member 710 according to a modification of the present embodiment.

### Description of Embodiments

### <<<<Outline of present embodiment>>>>

### <<First embodiment>>

According to a first embodiment,
provided is a cell state estimation system including:
an appearance information acquisition portion (for example, a camera 310, a captured image acquisition portion 340 described later, and the like) that acquires appearance information of a second cell (for example, a sensor cell described later or the like) different from a first cell (for example, a product cell described later or the like) to be cultured, in which at least a part of a culture medium providing a growth environment to the first cell provides a growth environment to the second cell;
a physical property measurement portion (for example, an analysis/measurement device 320, an analysis/measurement result acquisition portion 350, and the like) that measures at least one physical property of at least one of the culture medium that has provided a growth environment of the second cell and the second cell; and
an estimation reference information portion (for example, a state estimation portion 360, a state space table for estimation described later, or the like) that stores estimation reference information referred to for estimating a state of at least one of the first cell and the culture medium, in which the estimation of the state of the at least one is based on at least one of a result acquired by the appearance information acquisition portion and a result measured by the physical property measurement portion.

The cell state estimation system includes an appearance information acquisition portion, a physical property measurement portion, and an estimation reference information portion.

The appearance information acquisition portion acquires appearance information of a second cell different from the first cell to be cultured. The first cell is to be cultured. The second cell is different from the first cell. The second cell may be a cell to be cultured.

The culture medium is a medium that provides a growth environment to the cell. At least a part of a culture medium that provides a growth environment to the first cell is provided as a growth environment of the second cell. **In** this way, at least a part of the culture medium is shared by the first cells and the second cells. All of the culture medium provided to the first cells may be provided as a growth environment of the second cell.

The physical property measurement portion measures at least one physical property for at least one of a culture medium that has provided a growth environment of the second cell and the second cell. Physical properties related to both the culture medium that has provided a growth environment of the second cell and the second cell may be measured, or physical properties related to either one of them may be measured.

The estimation reference information portion stores estimation reference information. The estimation reference information is information to be referred to for estimating a state of at least one of the first cell and the culture medium. The estimation reference information portion may estimate both the state of the first cell and the state of the culture medium, or may estimate only one of the states.

The estimation is performed based on at least one of the result acquired by the appearance information acquisition portion and the result measured by the physical property measurement portion. The estimation may be performed based on both results, or may be performed based on either one of the results.

The appearance information acquisition portion acquires appearance information of the second cell different from the first cell. That is, the appearance information acquisition portion does not directly acquire the appearance information of the first cell. In addition, the physical property measurement portion measures physical properties related to a culture medium that has provided a growth environment of the second cell and physical properties related to the second cell. That is, the physical property measurement portion does not directly acquire the culture medium that has provided a growth environment of the first cell or physical properties related to the first cell.

That is,
the second cell is preferably a cell that functions as a sensor for estimating the state of the first cell.

Further, the second cell is preferably arranged at a position separated from the first cell.

The estimation reference information portion estimates the state of the first cell and the state of the culture medium from the appearance information of the second cell, the physical properties related to the culture medium that has provided a growth environment of the second cell, and the physical properties related to the second cell. That is, the estimation reference information portion indirectly determines the state of the first cell and the like from the state of the second cell and the like. With such a configuration, even if the growth environment of the first cell is inappropriate for observation, measurement, and the like, the state of the first cell and the like can be accurately determined by using the state of the second cell and the like.

In addition, it is possible to maintain the growth environment of the first cell clean without contaminating the growth environment of the first cell by observation, measurement, and the like.

### <<Second embodiment>>

A second embodiment further includes, in the first embodiment,
at least one first tank (for example, a cell culture tank 100 described later or the like) in which the first cells are immersed in a culture medium and stored;
at least one second tank (for example, a pseudo culture tank 110 described later or the like) in which the second cells are immersed in a culture medium and stored; and
a communication portion (for example, a pipe 200B, a pipe 200C, and an organ cell tank 120 described later, and the like) that communicates the first tank and the second tank such that the culture medium can flow between the first tank and the second tank.

### <<Third embodiment>>

A third embodiment further includes, in the second embodiment,
at least one third tank (for example, an organ cell tank 120 described later or the like) in which third cells (for example, feeder cells described later or the like) for growing the first cells are stored and the culture medium is flowable.

The culture medium preferably flows through the first tank, the second tank, and the third tank.

### <<Fourth embodiment>>

A fourth embodiment further includes, in the first embodiment,
a control portion (for example, an estimation device 300, a control device 330, and a pump 210 described later, and the like) that determines a flow amount of the culture medium based on a reference result of the estimation reference information.

### <<Fifth embodiment>>

A fifth embodiment further includes, in the first embodiment,
a control portion (for example, an estimation device 300 and a control device 330 described later, and the like) that determines a timing of replacing the culture medium based on a reference result of the estimation reference information.

### <<Sixth embodiment>>

A sixth embodiment further includes, in the first embodiment,
a control portion (for example, an estimation device 300 and a control device 330 described later, and the like) that determines a timing of adding nutrients to the culture medium based on a reference result of the estimation reference information is further included.

### <<<<Details of present embodiment>>>>

A cell growth estimation system 10 according to the present embodiment estimates the degree of growth of product cells from the degree of growth of sensor cells and the state of the culture medium providing a growth environment of the sensor cells while keeping a growth environment of the product cells clean. The cell growth estimation system 10 controls the state of liquid medium, such as adding necessary nutrients to the liquid medium, based on an estimation result of the liquid medium. The cell growth estimation system 10 estimates the degree of growth of product cells at that time, and predicts the degree of growth of product cells thereafter (in the future).

### <<<Object of cell growth estimation system 10>>>

Examples of the object of the cell growth estimation system 10 include product cells, sensor cells (canary cells), a culture medium (liquid medium), and feeder cells.

### <<Product cell>>

The product cell is to be cultured. For example, the product cell is preferably a cell constituting edible meat or the like. The product cell is not limited to one for food, and may be one that can be grown. The product cell is grown by culture. The degree of growth, health condition, and the like of product cell are also referred to as the state of the product cell.

### <<Sensor cell (canary cell) (alternative sensing cell)>>

The sensor cell is a cell for estimating the state of the product cell. In the cell growth estimation system 10, the state of the product cell is not directly acquired, but indirectly acquired (estimated) by the sensor cell.

The sensor cell is preferably a cell that does not proliferate. As the sensor cell, for example, a nerve cell, a Sertoli cell, or the like can be used as the sensor cell. In addition, the sensor cell can be the same cell as the product cell. As the sensor cell, for example, a muscle cell, a liver cell, or the like can be used as the sensor cell.

### <<Culture medium (liquid medium)>>

The culture medium is supplied to both the product cells and the sensor cells. The culture medium flows between the product cells and the sensor cells. The culture medium is shared by both the product cells and the sensor cells. The liquid culture medium is also referred to as a culture solution.

### <<Feeder cell>>

The feeder cells are cells for growing product cells. For example, the feeder cells can be cells of the lung, the intestine, the brain, the muscle, the skin, the genital tract, the stomach, and the like in addition to the liver, the kidney, and the like. The feeder cells grow product cells or improve the quality of product cells by a combination of these organs. Specifically, various endocrine factors (hormones and cytokines) released from the feeder cells are given to product cells to grow the product cells.

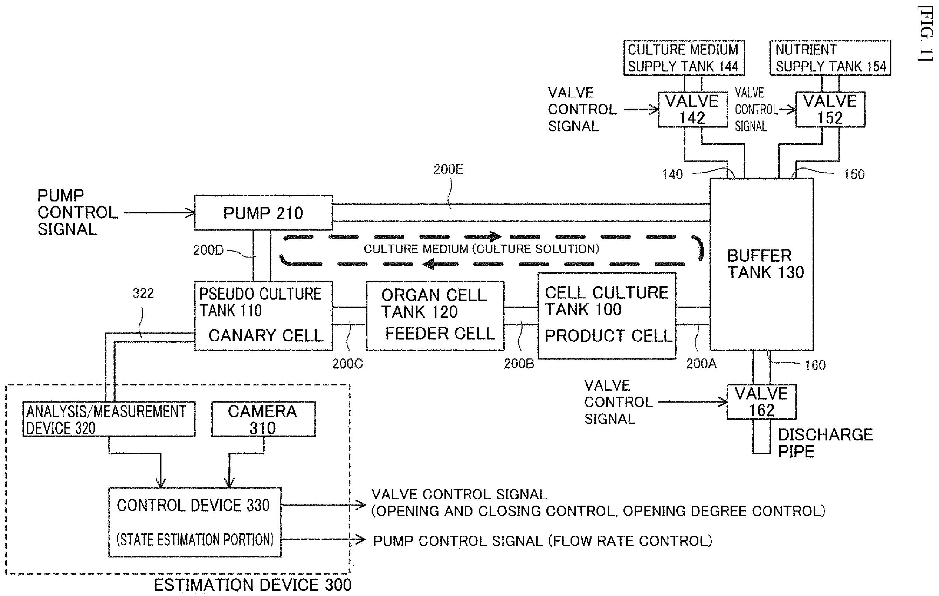

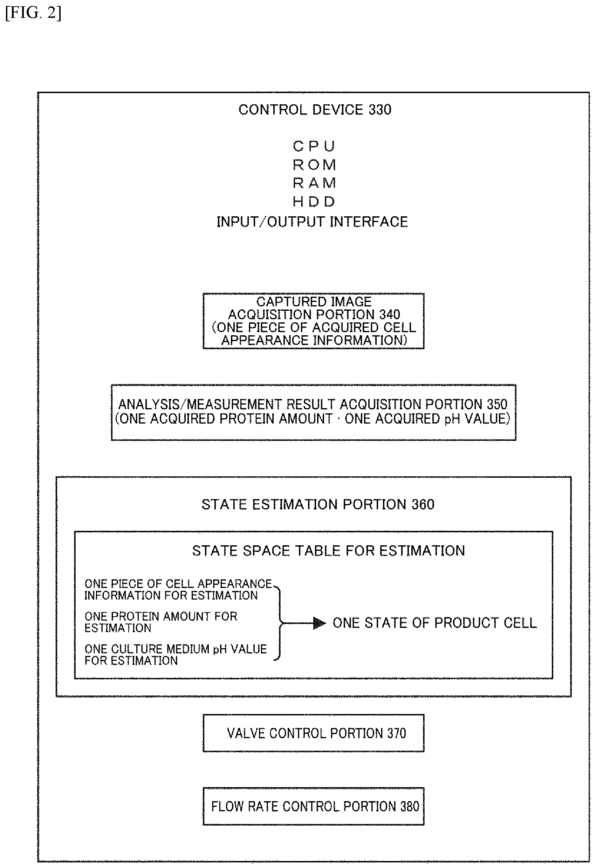

Hereinafter, embodiments will be described with reference to the drawings. Fig. 1 is a block diagram that illustrates an example of a cell state estimation system according to the present embodiment. Fig. 2 is a functional block diagram that illustrates a configuration of a control device 330 of the cell state estimation system according to the present embodiment.

### <<<Configuration of cell growth estimation system 10>>>

The cell growth estimation system 10 mainly includes a cell culture tank 100, a pseudo culture tank 110, an organ cell tank 120, a buffer tank 130, a pump 210, and an estimation device 300.

### <<Cell culture tank 100>>

The cell culture tank 100 is a tank for growing product cells. A culture medium is stored in the cell culture tank 100. The product cells are maintained in a state of being immersed in the culture medium.

The cell culture tank 100 has a certain shape and size. The cell culture tank 100 has, for example, a rectangular parallelepiped shape. The cell culture tank 100 may have a size sufficient for growth of product cells. The cell culture tank 100 may have a size capable of storing the product cells until reaching the final growth state without taking out the product cells in the growth process.

The cell culture tank 100 is formed of a material capable of visually recognizing the inside. The material of the cell culture tank 100 may be appropriately determined according to the growth environment of the product cells and the like. The cell culture tank 100 is formed of, for example, a resin such as glass or plastic. In the cell culture tank 100, the state of growth of product cells such as the amount of culture medium can be visually recognized. The cell culture tank 100 may have a visible window even if a part thereof is made of metal or the like.

The cell culture tank 100 has a lid body (not shown). The cell culture tank 100 is sealed with a lid body. The sealed state of the cell culture tank 100 is maintained by the lid body during the growth of product cells. The cell culture tank 100 can be kept clean by the sealed state.

In the cell culture tank 100, an amount of culture medium necessary for growing product cells is stored. In the cell culture tank 100, the culture medium is flowable. In the cell culture tank 100, the entire product cells are immersed in the culture medium. In the cell culture tank 100, a state in which the surface of the product cell is in contact with the culture medium is maintained.

The cell culture tank 100 includes a holding member (not shown) that holds product cells, a locking member (not shown), and the like. The product cells are held or locked without contacting a side wall (not shown) of the cell culture tank 100 or the like. The product cells can be smoothly grown in the cell culture tank 100.

### <Pipe 200A and pipe 200B>

A pipe 200A and a pipe 200B are connected to the cell culture tank 100.

The cell culture tank 100 has an opening (not shown) for the pipe 200A. The pipe 200A is connected to the cell culture tank 100. The cell culture tank 100 communicates with the buffer tank 130 through the pipe 200A. The culture medium flows in from the buffer tank 130. The culture medium is flowed by the pump 210 described later. The position and size of the opening of the pipe 200A may be appropriately determined.

The cell culture tank 100 has an opening (not shown) for the pipe 200B. The pipe 200B is connected to the cell culture tank 100. The cell culture tank 100 communicates with the organ cell tank 120 described later through the pipe 200B. The culture medium flows out to the organ cell tank 120. The position and size of the opening of the pipe 200B may be appropriately determined.

### <Flow of culture medium in cell culture tank 100>

The culture medium flows in from the buffer tank 130, flows in the cell culture tank 100, and flows out to the organ cell tank 120. The culture medium flows in through the pipe 200A and flows out through the pipe 200B. The culture medium always flows in the cell culture tank 100. The culture medium is smoothly supplied to the cell culture tank 100 to provide a growth environment suitable for the growth of product cells.

### <<Pseudo culture tank 110>>

The sensor cells are stored in the pseudo culture tank 110. The pseudo culture tank 110 is a tank for growing and observing the sensor cells. The culture medium is stored in the pseudo culture tank 110. Like the product cells, the sensor cells are maintained in a state of being immersed in the culture medium.

The pseudo culture tank 110 has a certain shape and size. The pseudo culture tank 110 has, for example, a rectangular parallelepiped shape. The pseudo culture tank 110 may have a size sufficient for growth and observation of sensor cells. The pseudo culture tank 110 may have a size capable of storing the sensor cells until reaching the final growth state of the product cells without taking out the sensor cells in the growth process.

The pseudo culture tank 110 is formed of a material, the inside of which can be observed. The material of the pseudo culture tank 110 may be appropriately determined according to the growth environment and observation environment of the sensor cells, and the like. The pseudo culture tank 110 is formed of, for example, a resin such as glass or plastic. Specifically, the pseudo culture tank 110 may be configured such that the state of the sensor cell can be imaged by a camera 310 described later.

In the pseudo culture tank 110, an amount of culture medium necessary for growing and maintaining sensor cells is stored. In the pseudo culture tank 110, the culture medium is flowable in the pseudo culture tank 110. In the pseudo culture tank 110, the entire sensor cells are immersed in the culture medium. In the pseudo culture tank 110, a state in which the surface of the sensor cell is in contact with the culture medium is maintained.

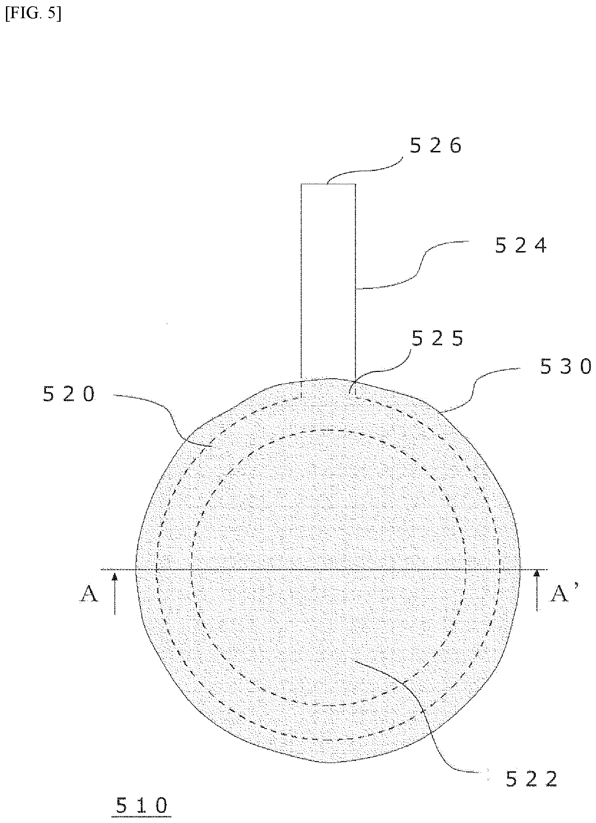

As illustrated in Fig. 5, the pseudo culture tank 110 includes a holding member 510 that holds the sensor cells. The sensor cells are held at a certain position by the holding member 510. By holding the sensor cells at a certain position, a certain part of the sensor cells can be stably imaged by the camera 310.

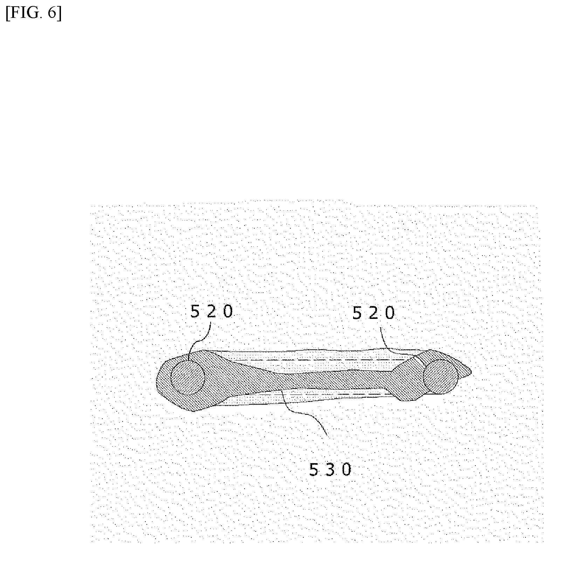

The holding member 510 will be described with reference to Figs. 5 and 6. Fig. 5 is a plan view that schematically illustrates the holding member 510. Fig. 6 is a cross-sectional view taken along line A-A' of the holding member 510 illustrated in Fig. 5.

The holding member 510 includes a frame member 520, a holding scheduled region 522, a rod member 524, and the like. The frame member 520 has a substantially annular shape. The holding scheduled region 522 is a region formed by being surrounded by the frame member 520. The holding scheduled region 522 has a substantially circular shape. The holding scheduled region 522 can be defined by the size and shape of the frame member 520. The rod member 524 has an elongated shape. The rod member 524 has a first end and a second end in the longitudinal direction. The frame member 520 is connected to the first end. The second end is held on a wall surface (not shown) of the pseudo culture tank 110 or the like. The holding member 510 can be positioned at a certain position of the pseudo culture tank 110. The frame member 520 is held in the pseudo culture tank 110 such that the holding scheduled region 522 faces the camera 310. Details will be described later.

The frame member 520 is a member that surrounds the entire holding scheduled region 522. The frame member 520 is made of a material having no cytotoxicity. The frame member 520 is at least partially covered with a cell population 530 of sensor cells, whereby the cell population 530 is held by the frame member 520. Therefore, the material of the frame member 520 is preferably a substrate having cell adhesion. By using a substrate having cell adhesion, the holding force can be enhanced. The material of the frame member 520 may be a substrate having no cell adhesion. Examples of the substrate that can be used include polymer materials or synthetic resin materials such as polyethylene, polypropylene, polyester, polyamide, polyimide, polyurethane, polycarbonate, and polypeptide, inorganic compounds such as stainless steel, apatite, and calcium phosphate, or natural materials such as sponge, cellulose, soy protein, polysaccharides, gelatin, egg white, mycelia, and decellularized tissue. The substrate is preferably polyimide or sponge from the viewpoint of cell adhesion.

In Fig. 6, a cross-sectional shape of the frame member 520 is substantially annular. The cross-sectional shape of the frame member 520 may be polygonal, substantially circular, substantially elliptical, or the like. The cross-sectional diameter of the frame member 520 (the maximum width of the frame member cross-sectional length orthogonal to the holding scheduled region surface) is not particularly limited. The lower limit value of the cross-sectional diameter of the frame member 520 is, for example, 0.1 µm or more, 1 µm or more, 3 µm or more, or 10 µm or more. The upper limit value of the cross-sectional diameter of the frame member 520 is, for example, 10 mm or less, 1 mm or less, or 100 µm or less.

The holding scheduled region 522 is a region formed by the inner part of the frame member 520, and is a region in which the cell population 530 in which a plurality of sensor cells are aggregated can be held. The holding scheduled region 522 may be a region surrounded by the frame member 520, and is preferably a region in which the inside of the frame member 520 is a space. The shape of the holding scheduled region 522 is substantially circular, but may be polygonal, substantially elliptical, or the like.

The area of the holding scheduled region 522 is large enough to immerse the frame member 520 in the culture medium. Specifically, for example, the area of the holding scheduled region 522 is 1,000,000 µm² or less, preferably 500 to 600,000 µm², more preferably 1,000 to 600,000 µm², and particularly preferably 1,000 to 6,000 µm². When the holding scheduled region 522 is in such a range, the cell population 530 can be held in the holding scheduled region at a high occupancy rate.

The rod member 524 is a rod-shaped member having an elongated shape. The rod member 524 has a first end 525 and a second end 526 in the longitudinal direction. The frame member 520 is connected to the first end 525. The second end 526 is gripped (held) by a gripping portion (not shown) provided with a gripping mechanism provided on a wall surface of the pseudo culture tank 110. The rod member 524 is formed of the same substrate as the frame member 520, but may be formed of another substrate. The rod member 524 is integrally molded with the frame member 520. The rod member 524 may be molded separately from the frame member 520. The rod member 524 is not particularly limited in shape as long as it can be gripped.

The gripping mechanism includes a drive motor for X-direction movement, a drive motor for Y-direction movement, a drive motor for Z-direction movement, a drive motor for inclination, and the like. The control device 330 issues a control signal to control the amount of movement of the drive motor for X-direction movement, the drive motor for Y-direction movement, and the drive motor for Z-direction movement, the inclination of the drive motor for inclination, and the like.

The movement, inclination, and the like of the rod member 524 (holding member 510) gripped by the gripping portion in the XYZ directions are controlled by the control device 330. With such control, the rod member 524 (holding member 510) can be easily handled. That is, the control device 330 can easily move the cell population 530 held by the holding member 510. As a result, since the holding scheduled region 522 can be adjusted to face the camera 310, a specific part of the cell population 530 can be easily shot with the camera 310.

The cell population 530 is an aggregate of membranous sensor cells that occupies at least a part of the holding scheduled region 522 and is held by the frame member 520. In Fig. 5, the cell population 530 occupies the entire holding scheduled region 522, but the cell population 530 may occupy a part of the holding scheduled region 522. The cell population 530 occupies preferably 50% or more, more preferably 80% or more, still more preferably 90% or more of the area of the holding scheduled region 522, and most preferably occupies the entire holding scheduled region 522.

At least a part of the peripheral edge of the cell population 530 is in contact with the frame member 520. Chemical bonding between the peripheral edge of the cell population 530 and the frame member 520 is not necessarily required. As illustrated in Fig. 6, in a predetermined cross section, the entire outer periphery of the frame member 520 may be covered with a part of the cell population 530 (see the left side of Fig. 6), or a part of the outer periphery of the frame member 520 may be covered with a part of the cell population 530 (see the right side of Fig. 6). From the viewpoint of enhancing the holding force, it is preferable that the entire outer periphery of the frame member 520 is covered with a part of the cell population 530 in a predetermined cross section.

As illustrated in Fig. 6, a portion of the cell population 530 occupying the holding scheduled region 522 floats in the air. The phrase "floats in the air" means that one surface and/or the other surface of the portion of the cell population 530 occupying the holding scheduled region 522 is exposed, and is in a state where a space without an interference object exists on one surface side and the other surface side of the cell population 530. Accordingly, for example, since there is no obstacle such as a scaffold when observing cells with the camera 310 or the like, it is possible to provide a holding member capable of holding cells without hindering cell observation by the scaffold to which the cells adhere, and a cell holding method thereof.

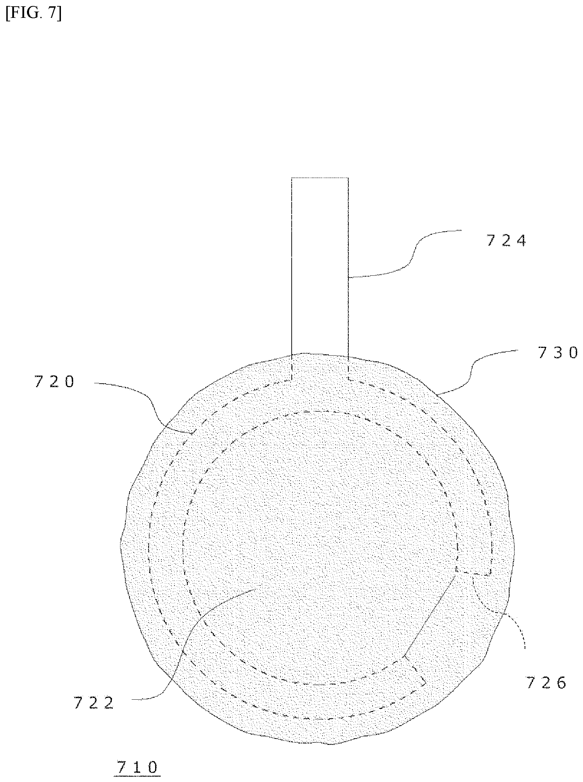

A modification of the holding member will be described with reference to Fig. 7. Fig. 7 is a plan view that schematically illustrates a modification of the holding member. The basic configuration of the holding member 710 according to the modification is similar to that of the present embodiment. Hereinafter, a configuration different from that of the present embodiment will be described.

A frame member 720 according to the modification is a member having a shape in which a part of the frame member 520 is notched. The frame member 720 surrounds a part of a holding scheduled region 722. As described above, when the frame member 720 has an open structure (a structure provided with a notch), a line connecting end points of the open structure portions at the shortest distance is regarded as an inner part of the frame member, and the holding scheduled region 722 is defined. A rod member 724 is the same as the rod member 524.

According to the holding member 710 of the modification, in a notch portion 726 of the frame member 720, shooting of the cell population 530 with the camera 310 and shooting from a direction parallel to the holding scheduled region 722 with the camera 310 are facilitated, so that it is possible to provide a holding member capable of holding cells without hindering cell observation by the scaffold to which the cells adhere, and a cell holding method thereof.

The sensor cells may be locked at a certain position by a member having translucency such as a preparation. By locking the sensor cells at a certain position, a certain part of the sensor cells can be stably imaged by the camera 310.

A method of holding cells in the holding member 510 (holding member 710) will be described. The culture medium, the sensor cells, and the frame member 520 (frame member 720) are prepared. The frame member 520 (frame member 720) is immersed in the culture medium containing the sensor cells. The immersion time of the frame member 520 (frame member 720) can be, for example, 1 hour or more, 2 hours or more, 5 hours or more, 10 hours or more, or 24 hours or more.

When the frame member 520 (frame member 720) is immersed, the holding member 510 (holding member 710) in which the membranous cell population 530 (cell population 730) is formed in the holding scheduled region is obtained. According to such a cell holding method, the cell population 530 (cell population 730) can be easily held by the holding member.

With the holding member prepared by the above method, the cells can be held without hindering cell observation by the scaffold to which the cells adhere, so that operation of the cell population in microunits is facilitated. The holding member may be used in the prepared culture vessel or may be taken out and used.

A certain amount of culture medium is stored in the pseudo culture tank 110. In the pseudo culture tank 110, a growth environment of the sensor cells is provided by the culture medium. In the pseudo culture tank 110, the culture medium is flowable. At least a part of the culture medium flowing into the pseudo culture tank 110 is the culture medium flowing out of the cell culture tank 100. The culture medium in the pseudo culture tank 110 is shared with the culture medium in the cell culture tank 100. By sharing the culture medium between the cell culture tank 100 and the pseudo culture tank 110, a growth environment common to the growth environment of the product cells is provided to the sensor cells.

### <Pipe 200C and pipe 200D>

A pipe 200C and a pipe 200D are connected to the pseudo culture tank 110.

The pseudo culture tank 110 has an opening (not shown) for the pipe 200C. The pipe 200C is connected to the pseudo culture tank 110. The pseudo culture tank 110 communicates with the organ cell tank 120 through the pipe 200C. The culture medium flows in from the organ cell tank 120. The culture medium is flowed by the pump 210 described later. The position and size of the opening of the pipe 200C may be appropriately determined.

The pseudo culture tank 110 has an opening (not shown) for the pipe 200D. The pipe 200D is connected to the pseudo culture tank 110. The pseudo culture tank 110 communicates with the pump 210 described later through a pipe 200D. The culture medium flows out to the pump 210. The position and size of the opening of the pipe 200D may be appropriately determined.

### <Flow of culture medium in pseudo culture tank 110>

The culture medium flows in from the organ cell tank 120, flows in the pseudo culture tank 110, and flows out to the pump 210. The culture medium flows in through the pipe 200C and flows out through the pipe 200D. The culture medium always flows in the pseudo culture tank 110. The culture medium is smoothly supplied to the pseudo culture tank 110 to provide a growth environment suitable for growth and maintenance of sensor cells.

### <<Growth environment in pseudo culture tank 110>>

The culture medium is flowed by the pump 210. Specifically, the culture medium is refluxed between the cell culture tank 100, the pseudo culture tank 110, the organ cell tank 120, and the buffer tank 130. In the example illustrated in Fig. 1, the culture medium is refluxed in the order of the cell culture tank 100, the organ cell tank 120, the pseudo culture tank 110, the pump 210, and the buffer tank 130. Due to the reflux of the culture medium, the culture medium is shared by both the cell culture tank 100 and the pseudo culture tank 110. By sharing the culture medium, a growth environment common to the growth environment of the product cells in the cell culture tank 100 is provided to the sensor cells in the pseudo culture tank 110.

### <<Organ cell tank 120>>

The organ cell tank 120 is a tank for storing feeder cells. A culture medium is stored in the organ cell tank 120. Like the product cells and the sensor cells, the feeder cells are maintained in a state of being immersed in the culture medium.

In the organ cell tank 120, the culture medium is flowable. In the organ cell tank 120, the entire feeder cells are immersed in the culture medium. In the organ cell tank 120, a state in which the surface of the feeder cell is in contact with the culture medium is maintained.

The organ cell tank 120 includes a holding member (not shown) that holds feeder cells, a locking member (not shown), and the like. The feeder cells are held or locked without contacting a side wall (not shown) of the organ cell tank 120 or the like. In the organ cell tank 120, the feeder cells can be stored in a desired environment.

### <Pipe 200B and pipe 200C>

The pipe 200B and the pipe 200C are connected to the organ cell tank 120. The organ cell tank 120 communicates with the cell culture tank 100 through the pipe 200B. The organ cell tank 120 communicates with the pseudo culture tank 110 through the pipe 200C. The culture medium flows in from the cell culture tank 100, flows in the organ cell tank 120, and flows out to the pseudo culture tank 110.

### <<Buffer tank 130>>

The culture medium is stored in the buffer tank 130. A pipe 200E and the pipe 200A are connected to the buffer tank 130. The buffer tank 130 communicates with the pump 210 through the pipe 200E. The buffer tank 130 communicates with the cell culture tank 100 through the pipe 200A. The culture medium is refluxed via the buffer tank 130.

The buffer tank 130 has a culture medium supply port 140. A culture medium supply tank 144 is connected to the culture medium supply port 140 via a valve 142. Opening and closing of the valve 142 is controlled by the control device 330. A new culture medium can be supplied from the culture medium supply tank 144 to the buffer tank 130. It is preferable to supply a new culture medium through a filter (not shown) that does not allow fungi to pass through. The buffer tank 130 can be kept clean.

The buffer tank 130 has a nutrient supply port 150. A nutrient supply tank 154 is connected to the nutrient supply port 150 via a valve 152. Opening and closing of the valve 152 is controlled by the control device 330. The necessary nutrients can be supplied from the nutrient supply tank 154 to the buffer tank 130. It is preferable to supply necessary nutrients through a filter (not shown) that does not allow fungi to pass through. The buffer tank 130 can be kept clean.

The buffer tank 130 has a culture medium discharge port 160. A part of the culture medium can be discharged from the culture medium discharge port 160 via the valve 162. Opening and closing of the valve 162 is controlled by the control device 330.

For example, a predetermined amount (for example, half the amount of the buffer tank 130) of culture medium is discharged from the culture medium discharge port 160, and the same amount of new culture medium is introduced from the culture medium supply tank 144 via the culture medium supply port 140 together with the discharge. In this way, a part of the culture medium can be replaced. Specifically, the culture medium is replaced on condition that the pH value of the sampled culture medium or the amount of protein contained in the sampled culture medium falls below a predetermined threshold.

### <<Pump 210>>

The pump 210 includes an electric motor (not shown). The culture medium is caused to flow by the electric motor. The flow rate of the culture medium per unit time is controlled by the rotation speed of the electric motor. The pump 210 is driven by a control signal from the control device 330. The control signal includes information indicating the rotation speed of the electric motor.

The pipe 200D and the pipe 200E are connected to the pump 210. The pump 210 communicates with the pseudo culture tank 110 through the pipe 200D. The pump 210 communicates with the buffer tank 130 through the pipe 200C. The culture medium flows into the pump 210 from the pseudo culture tank 110, and flows out from the pump 210 to the buffer tank 130 by driving force of the pump 210.

The culture medium can be refluxed by driving the pump 210. Specifically, in the example illustrated in Fig. 1, the culture medium is refluxed in the order of the cell culture tank 100, the organ cell tank 120, the pseudo culture tank 110, the pump 210, and the buffer tank 130.

### <<Estimation device 300>>

The estimation device 300 includes the camera 310, an analysis/measurement device 320, and the control device 330. As described above, the growth environment common to the growth environment of the product cells in the cell culture tank 100 is provided to the sensor cells in the pseudo culture tank 110. Therefore, the state of the product cell can be estimated from the fact that the state of the sensor cell is equal to the state of the product cell. The estimation device 300 estimates the state of the product cell, controls opening and closing of the valve 142, the valve 152, and the valve 162 of the buffer tank 130, and controls the flow rate of the culture medium by the pump 210.

### <Camera 310>

The camera 310 images the sensor cell to acquire appearance information of the sensor cell. From the appearance information of the sensor cell, the health condition of the sensor cell can be determined. The appearance information of the sensor cell may be information of a narrow region of the sensor cell or information of a wide region of the sensor cell. The appearance information may be any information as long as the health condition of the sensor cell can be determined.

The camera 310 includes an imaging element such as a CCD element or a CMOS element. The imaging element may be any imaging element suitable for imaging the appearance such as the surface of the sensor cell. The imaging element has a predetermined resolution. The resolution may be any resolution necessary for estimating the health condition of the sensor cell.

The camera 310 may have an objective lens (not shown). The appearance of the sensor cell is imaged at a desired magnification by the objective lens. The magnification may be a magnification necessary for determining the health condition of the sensor cell.

Furthermore, a lighting device (not shown) may be provided. Brightness required to determine the health condition of the sensor cell can be achieved. The lighting device illuminates the surface of the sensor cell. It is preferable that the lighting device does not change the growth environment such as the temperature of the sensor cells and the culture medium. The lighting device emits light having a wavelength necessary for imaging the appearance, such as white light. Brightness and wavelength that do not affect the growth of sensor cells are preferable.

Imaging by the camera 310 is controlled by a control signal from the control device 330. An imaging condition is determined by the control signal, imaging is started by the control signal, and imaging is terminated by the control signal.

The image captured by the camera 310 may be a single still image or a moving image. The image captured by the camera 310 may be a plurality of still images captured at predetermined timings. The image captured by the camera 310 may be any image as long as the health condition of the sensor cell can be determined.

### <Analysis/measurement device 320>

The analysis/measurement device 320 is connected to the pseudo culture tank 110 via a sampling pipe 322. The analysis/measurement device 320 analyzes and measures a culture medium sampled from the pseudo culture tank 110. The analysis/measurement device 320 mainly analyzes and measures a culture medium that has provided a growth environment of the sensor cells. With this configuration, the analysis/measurement device 320 can analyze and measure the culture medium while hardly affecting the growth environment of the product cells.

The analysis/measurement device 320 includes an optical sensor (not shown), a potential sensor (not shown), and the like.

### <Optical sensor (spectroscopic analysis)>

The optical sensor emits light from a light source and irradiates the sampled culture medium. The analysis/measurement device 320 quantifies the absorbance of the sampled culture medium. The analysis/measurement device 320 can acquire the amount of protein contained in the sampled culture medium. The analysis/measurement device 320 acquires the amount of protein contained in the culture medium that has provided a growth environment of the sensor cells. The analysis/measurement device 320 may measure the concentration of glucose.

### <Potential sensor>

The potential sensor quantifies the potential difference of the sampled culture medium. The analysis/measurement device 320 can acquire a pH value of the sampled culture medium. The analysis/measurement device 320 acquires a pH value of the culture medium that has provided a growth environment of the sensor cells.

### <Others>

The analysis/measurement device 320 may measure the temperature of the culture medium. The analysis/measurement device 320 may acquire a parameter that changes according to the growth of the product cell or the sensor cell. The parameter to be acquired may be any parameter that can estimate the state of the product cell from the value of the parameter.

For example, the analysis/measurement device 320 may detect contamination (accumulation of metabolites) of a culture solution to obtain data for instructing replacement of the culture solution or instructing operation of a dialyzer, and predict the replacement time from the obtained data. Specifically, the analysis/measurement device 320 can predict nutrient starvation of cells, changes caused by excessive metabolites, and the like.

In addition, the analysis/measurement device 320 may detect components of metabolites. The analysis/measurement device 320 may determine the deterioration state and the health condition of the culture medium.

### <Control device 330>

As illustrated in Fig. 2, the control device 330 mainly includes a central processing unit (CPU), a read only memory (ROM), a random access memory (RAM), a hard disk drive (HDD), an input/output interface, and the like (all not shown). The CPU, the ROM, the RAM, the HDD, the input/output interface, and the like constitute a captured image acquisition portion 340, an analysis/measurement result acquisition portion 350, a state estimation portion 360, a valve control portion 370, and a flow rate control portion 380.

### <Captured image acquisition portion 340>

The captured image acquisition portion 340 is electrically connected to the camera 310 described above via the input/output interface. The captured image acquisition portion 340 outputs a control signal indicating an imaging condition, a control signal for starting imaging, a control signal for terminating imaging, and the like to the camera 310 at predetermined timings.

The captured image acquisition portion 340 acquires appearance information of the sensor cell from the imaging signal output from the camera 310. The captured image acquisition portion 340 receives the imaging signal output from the camera 310, and forms a captured image as appearance information. The appearance information is sequentially stored in the HDD or the like.

The appearance information of the sensor cell is accumulated in the HDD or the like. From the appearance information of the sensor cell, a change in the health condition of the sensor cell can be determined.

### <Analysis/measurement result acquisition portion 350>

The analysis/measurement result acquisition portion 350 is electrically connected to the analysis/measurement device 320 described above (optical sensor or potential sensor) via the input/output interface. The analysis/measurement result acquisition portion 350 acquires the amount of protein (concentration of glucose) in the culture medium from an output signal of the optical sensor, and acquires the pH value of the culture medium from an output signal of the potential sensor.

### <State estimation portion 360>

The state estimation portion 360 estimates the health condition of the product cell from the appearance information of the sensor cell, the amount of protein (concentration of glucose) in the culture medium, and the pH value of the culture medium.

<Acquired cell appearance information, acquired protein amount (acquired glucose concentration), acquired pH value>

For convenience, names are given as follows. The appearance information of the sensor cell acquired by the captured image acquisition portion 340 is referred to as acquired cell appearance information. The amount of protein (glucose concentration) in the culture medium acquired by the analysis/measurement result acquisition portion 350 is referred to as an acquired protein amount (acquired glucose concentration). The pH value of the culture medium acquired by the analysis/measurement result acquisition portion 350 is referred to as an acquired pH value.

### <State space table for estimation>

The state estimation portion 360 includes a state space table for estimation. The state space table for estimation is a table referred to for estimating one state of the product cell from one piece of acquired cell appearance information, one acquired protein amount (one acquired glucose concentration), and one acquired pH value.

In the state space table for estimation, a plurality of states (health condition and growth state) of the product cell are stored in association with a plurality of pieces of cell appearance information for estimation of sensor cells, a plurality of protein amounts for estimation (a plurality of glucose concentrations for estimation) in the culture medium, and a plurality of culture medium pH values for estimation of the culture medium. Specifically, in the state space table for estimation, one piece of cell appearance information for estimation, one protein amount for estimation, and one culture medium pH value for estimation are associated with one state of the product cell, and a correspondence relationship therebetween is stored.

### <Example of state space table for estimation>

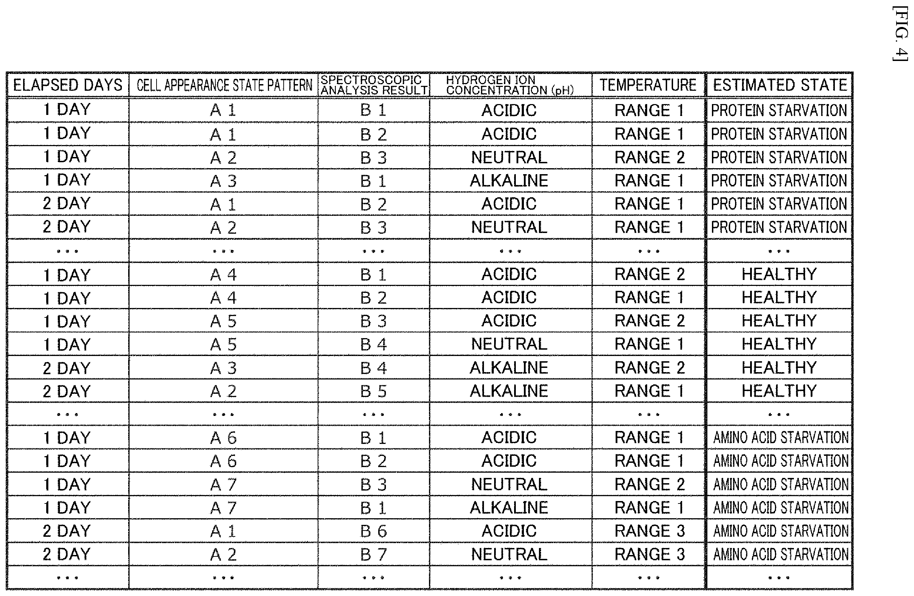

Fig. 4 is an example of the state space table for estimation. In the state space table for estimation illustrated in Fig. 4, "cell appearance information for estimation" is indicated as "cell appearance state pattern", "protein amount for estimation" is indicated as "spectroscopic analysis result", "culture medium pH value for estimation" is indicated as "hydrogen ion concentration (pH)", and "one state of the product cell" is indicated as "estimated state".

In addition, the state space table for estimation illustrated in Fig. 4 also includes the number of days elapsed (elapsed days) from the start of growth and the temperature of the culture medium. By using these elapsed days, temperatures, and the like, the state of the product cell can be determined more accurately.

### <Cell appearance information for estimation, protein amount for estimation (glucose concentration for estimation), culture medium pH value for estimation>

The plurality of pieces of cell appearance information for estimation is a plurality of appearance information of the sensor cell corresponding to each of a plurality of states of the product cell, which is acquired in advance in a preliminary experiment. The plurality of protein amounts for estimation (the plurality of glucose concentrations for estimation) is a plurality of culture medium protein amounts (a plurality of glucose concentrations for estimation) corresponding to each of a plurality of states of growth of product cells, which is acquired in advance in a preliminary experiment. The plurality of culture medium pH values for estimation is a plurality of culture medium pH values corresponding to each of the plurality of states of the product cell, which is acquired in advance in a preliminary experiment.

The state estimation portion 360 refers to the state space table for estimation and selects one piece of cell appearance information for estimation closest to one piece of acquired cell appearance information among the plurality of pieces of cell appearance information for estimation.

The state estimation portion 360 refers to the state space table for estimation and selects one protein amount for estimation (one glucose concentration for estimation) closest to one acquired protein amount (one acquired glucose concentration) among the plurality of protein amounts for estimation (the plurality of glucose concentrations for estimation).

The state estimation portion 360 refers to the state space table for estimation and selects one culture medium pH value for estimation closest to one acquired pH value among the plurality of culture medium pH values for estimation.

The state estimation portion 360 determines one state of the product cell by referring to the state space table for estimation from the selected one piece of cell appearance information for estimation, one protein amount for estimation (one glucose concentration for estimation), and one culture medium pH value for estimation. One state determined by referring to the state space table for estimation is determined as the state of the product cell.

In this way, the state estimation portion 360 can determine one state of the product cell from one acquired cell appearance information, one acquired protein amount (one acquired glucose concentration), and one acquired pH value.

### <Specific Example 1 of state estimation>

For example,
when one culture medium protein amount for estimation is large (protein concentration is high), and
one piece of cell appearance information for estimation is unhealthy, and
one culture medium pH value for estimation is 6 (weakly acidic),
the state estimation portion 360 determines that the product cell at that time is in an unhealthy condition due to excessive metabolites (lactic acid).

### <Specific Example 2 of state estimation>

Further,
when one culture medium protein amount for estimation is small (protein concentration is low), and
one piece of cell appearance information for estimation is unhealthy, and
one culture medium pH value for estimation is 7 (neutral),
the state estimation portion 360 determines that the product cell at that time is in an unhealthy condition due to a lack of protein.

### <Prediction of subsequent state of product cell>

By sequentially determining the state of the product cell described above at predetermined timings, a temporal change in the state of the product cell can be acquired. From the temporal change in the state of the product cell, the subsequent state of the product cell can be estimated. For example, it is possible to estimate a time point at which the growth of the product cell is completed, a possibility that the product cell becomes unhealthy later, and the like.

### <Difference between state of product cell and state of sensor cell>

In the example described above, the state of the product cell is determined or estimated assuming that the state of the sensor cell is equal to the state of the product cell. However, the growth environment of the product cell is different from the growth environment of the sensor cell. There may be a difference between the state of the product cell and the state of the sensor cell. Therefore, by also storing the information on the difference between the state of the product cell and the state of the sensor cell in the state space table for estimation, it is possible to determine or estimate one state of the product cell even when a difference occurs.

### <Valve control portion 370>

The valve control portion 370 controls opening and closing of the valve 142, the valve 152, and the valve 162 from the determined health condition of the product cell. By sending an opening control signal or a closing control signal, these valves are opened or closed.

Not only opening and closing but also opening degrees of these valves may be controlled. A rapid change in the growth environment of the cell culture tank 100 and the pseudo culture tank 110 due to a rapid change in the buffer tank 130 can be prevented.

### <Flow rate control portion 380>

The flow rate control portion 380 controls the flow rate of the pump 210 from the determined health condition of the product cell. The flow rate control portion 380 outputs a signal indicating the flow rate of the culture medium. An electric motor (not shown) of the pump 210 rotates at a rotation speed according to the flow rate.

Feedback control can be performed by the valve control portion 370 and the flow rate control portion 380.

### <<State determination processing of product cell by state estimation portion 360>>

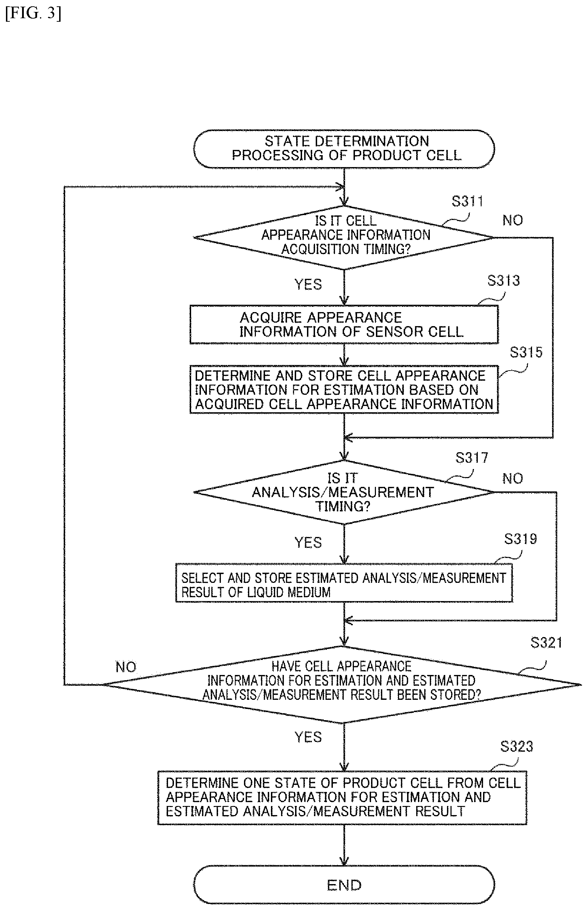

The state determination processing of the product cell will be described with reference to Fig. 3. First, a processor of the state estimation portion 360 determines whether or not it is timing to acquire cell appearance information (step S311).

When the processor of the state estimation portion 360 determines that it is the timing to acquire cell appearance information (YES), the processor acquires the appearance information of the sensor cell by the camera 310 (step S313).

Next, the processor of the state estimation portion 360 determines and stores the cell appearance information for estimation based on the cell appearance information acquired in the processing of step S313 (step S315).

When the processor of the state estimation portion 360 determines that it is not the timing to acquire cell appearance information in the determination of step S311 (NO) or executes the processing of step S315, the processor determines whether or not it is timing to perform analysis/measurement (step S317).

When the processor of the state estimation portion 360 determines that it is the timing to perform analysis/measurement (YES), the processor selects and stores the estimated analysis/measurement result of the liquid medium of the analysis/measurement device 320 (step S319).

When the processor of the state estimation portion 360 determines that it is not the timing to perform analysis/measurement in the determination of step S317 (NO) or executes the processing of step S319, the processor determines whether or not the cell appearance information for estimation and the estimated analysis/measurement result have been stored (step S321).

When the processor of the state estimation portion 360 determines that the cell appearance information for estimation and the estimated analysis/measurement result have been stored (YES), the processor determines one state of the product cell from the cell appearance information for estimation and the estimated analysis/measurement result (step S323), and ends this subroutine.

When the processor of the state estimation portion 360 determines that the cell appearance information for estimation and the estimated analysis/measurement result have not been stored (NO), the process returns to step S311.

### <<<Order, arrangement (parallel/series), number, and the like of each tank>>>

In the example described above, a configuration in which the cell culture tank 100, the organ cell tank 120, the pseudo culture tank 110, the pump 210, and the buffer tank 130 are arranged in this order has been shown, but the order is not limited thereto. For example, the pump 210 may be arranged immediately before the cell culture tank 100. The culture medium in the buffer tank 130 can be quickly supplied to the cell culture tank 100.

In the example described above, a configuration in which the cell culture tank 100, the pseudo culture tank 110, the organ cell tank 120, the buffer tank 130, and the pump 210 are arranged one by one has been shown, but the number is not limited thereto. By arranging a plurality of cell culture tanks 100, the number of product cells to be grown can be increased. By arranging a plurality of pseudo culture tanks 110, it is possible to more accurately determine the state of the product cell without individual differences of sensor cells or the like. By arranging a plurality of buffer tanks 130, it is possible to accurately control the ratio between culture medium and nutrients by separating the buffer tanks 130 into those for the culture medium and those for the nutrients.

In the example described above, a configuration in which the cell culture tank 100, the pseudo culture tank 110, the organ cell tank 120, the buffer tank 130, and the pump 210 are arranged in series has been shown, but the arrangement is not limited thereto. A plurality of cell culture tanks 100 may be arranged in parallel. It is possible to make effects such as the health condition of the product cell less likely to be exerted on each other. In addition, a plurality of rows of the cell culture tank 100, the pseudo culture tank 110, the organ cell tank 120, and the pump 210 arranged in series may be prepared, and arranged in parallel by sharing only the buffer tank 130. It is possible to make effects the state of the product cells, the sensor cells, and the feeder cells less likely to be exerted to other rows. In this manner, the series and the parallel can be arranged in a mixed manner.

In addition, the tanks may be branched or bypassed on the way. For example, the cell culture tank 100 may be connected to both the organ cell tank 120 and the buffer tank 130. A part of the culture medium flowing out of the cell culture tank 100 can be supplied to the organ cell tank 120, and the remaining culture medium flowing out of the cell culture tank 100 can be returned to the buffer tank 130.

### <<<<Range of embodiment>>>>

As described above, the present embodiment has been described. However, the description and the drawings constituting a part of the present disclosure should not be understood to be limiting. The present invention includes various embodiments and the like not described herein.

### Reference Signs List

- 10: Cell growth estimation system
- 100: Cell culture tank
- 110: Pseudo culture tank
- 120: Organ cell tank
- 300: Estimation device
- 310: Camera
- 320: Analysis/measurement device
- 340: Captured image acquisition portion
- 350: Analysis/measurement result acquisition portion
- 360: State estimation portion
- 510: Holding member
- 520: Frame member
- 522: Holding scheduled region
- 524: Rod member
- 525: First end
- 526: Second end
- 530: Cell population
- 710: Holding member
- 722: Holding scheduled region
- 724: Rod member
- 726: Notch portion
- 730: Cell population

## Claims

1. A cell state estimation system comprising:
an appearance information acquisition portion that acquires appearance information of a second cell different from a first cell to be cultured, wherein at least a part of a culture medium providing a growth environment to the first cell provides a growth environment to the second cell;
a physical property measurement portion that measures at least one physical property of at least one of the culture medium that has provided a growth environment of the second cell and the second cell; and
an estimation reference information portion that stores estimation reference information referred to for estimating a state of at least one of the first cell and the culture medium, wherein the estimation of the state of at least one is based on at least one of a result acquired by the appearance information acquisition portion and a result measured by the physical property measurement portion.

2. The cell state estimation system according to claim 1, further comprising:
at least one first tank in which the first cells are immersed in a culture medium and stored;
at least one second tank in which the second cells are immersed in a culture medium and stored; and
a communication portion that communicates the first tank and the second tank such that the culture medium can flow between the first tank and the second tank.

3. The cell state estimation system according to claim 2, further comprising at least one third tank in which third cells for growing the first cells are stored and the culture medium is flowable.

4. The cell state estimation system according to claim 1, further comprising a control portion that determines a flow amount of the culture medium based on a reference result of the estimation reference information.

5. The cell state estimation system according to claim 1, further comprising a control portion that determines a timing of replacing the culture medium based on a reference result of the estimation reference information.

6. The cell state estimation system according to claim 1, further comprising a control portion that determines a timing of adding nutrients to the culture medium based on a reference result of the estimation reference information.
